# EUROPEAN PATENT SPECIFICATION

(11) **EP 0 543 842 B1**
(45) Date of publication and mention of the grant of the patent: **31.01.1996**
(21) Application number: 91914015.2
(22) Date of filing: 22.07.1991
(51) Int. Cl.: G01N 33/543, G01N 33/58, G01N 21/77

(54) **ANALYTICAL DEVICE**
ANALYTISCHES GERÄT
DISPOSITIF ANALYSEUR

(30) Priority: 18.08.1990 GB 9018195
(43) Date of publication of application: 02.06.1993
(73) Proprietor: FISONS plc, Ipswich Suffolk IP1 1QH (GB)
(72) Inventor: DAVIES, Robert, John, Cambridge CB5 8HS (GB)
(74) Representative: Jones, Stephen Anthony
(86) International application number: GB9101221
(87) International publication number: WO9203730

(56) References cited:
- EP-A- 0 205 236
- EP-A- 0 212 989
- WO-A-90/06503
- WO-A-91/06862
- Patent Abstracts of JapAn, vol. 12, no. 56 (C-477)[2903], 19 ebruary 1988, & JP, A, 62201815 (TAKEDA CHEM IND LTD) 5 September 1987, see the abstract
- Chemical Abstracts, vol. 109, 7 November 1988, Columbus, Ohio, US; see page 387, abstract no. 166861a, & JP, A, 6315166 (TOSHIBA CORP.) 22 January 1988

## Description

This invention relates to the determination of biologically active molecules by immunoassay, in particular to devices of the kind known as biosensors which are used in such determinations.

Immunoassay techniques involve the formation of a complex between the species under test (the analyte) and another species to which it will bind specifically (a 'specific binding partner'). The extent to which complex formation occurs depends on the amount of analyte present.

In biosensor devices, the specific binding partner can be immobilised on the surface of the device and complex formation, and hence the analyte concentration, is monitored as a change in the properties of that surface, eg the optical properties.

A disadvantage of known immunoassay systems stems from the fact that the changes which occur on formation of the complex are relatively small and hence difficult to detect.

One way in which this problem may be overcome is by labelling the specific binding partner with an enzyme which acts on a substrate to produce a product with a high refractive index or strong colour. This effect amplifies the response of the device to the presence of analyte, increasing the sensitivity and hence lowering the detection limit.

In practice, however, the enzyme label and the substrate must be kept apart until complex formation occurs and hitherto this has been difficult to achieve in a biosensor device. Fabrication of the sensor such that the enzyme and its substrate are on separate areas, or surfaces, could be employed, but would require more elaborate fabrication methods and could impair sensor performance due to the considerable time taken for intimate mixing of substrate and enzyme.

WO-A-9 006 503 discloses a biosensor with an immobilized first binding partner and a second binding partner labelled with a chromophore or a fluorophore.

EP-A-0 212 989 discloses the use of liposomes to encapsulate labelled species and also means to lyse the liposomes during the assay. This document further discloses the use of these liposomes in a multilayered element.

However, EP-A-0 212 989 neither teaches that the multilayer should be included in a biosensor in order to perform an immunoassay nor that the encapsulated labelled species could be substituted by the substrate for an enzyme.

EP-A-0 205 236 refers to optic waveguide biosensors.

We have now devised a biosensor device in which an enzyme-substrate interaction may be used to amplify the response of the device to the presence of analyte.

According to the invention there is provided a biosensor for the detection of an analyte in solution using enzyme-substrate interaction for response amplification, the biosensor having a coating layer comprising an immobilised first specific binding partner for the analyte, an enzyme-labelled reagent being either a second specific binding partner for the analyte or a specific binding partner for the first specific binding partner, and a substrate for the enzyme, the substrate being encapsulated in a liposome.

The biosensor according to the invention is advantageous primarily in that it is more sensitive than other, known devices. This enables lower concentrations of analyte to be detected and/or analyte concentrations to be determined with greater accuracy. The device is nonetheless easy to use, measurements generally being made simply by bringing the sample into contact with the device, no further reagents being necessary.

In use, the enzyme and substrate are maintained separate, by virtue of the substrate being encapsulated in liposomes, until the device is brought into contact with a sample containing the analyte. Subsequently, as the sample is absorbed into the coating layer, either a 'sandwich' complex is formed between the enzyme-labelled reagent (where this a second specific binding partner for the analyte), the analyte and the first specific binding partner, or competitive binding of the alyte and labelled reagent (where this is a specific binding partner for the first specific binding partner, eg labelled analyte) with the first specific binding partner. In either case, the enzyme label is brought into the vicinity of substrate released from the liposomes and the enzyme-substrate interaction occurs.

Liposomes encapsulating the substrate may be prepared by any of the methods known in the art for this purpose. In general, liposomes of the type designated Reversed Phase Evaporated Vesicles (REV) are preferred since they have a relatively large aqueous core and low permeability.

The coating layer may take various forms, the principal requirements being that it be permeable to the sample.

The coating layer may suitably be a layer of gel, eg polyacrylamide gel, or a suitable polysaccharide, such as agarose or dextran.

The immobilised and enzyme-labelled specific binding partners may be similar, if the analyte has two similar epitopes, or they may be different, if the analyte has two different epitopes.

Although the enzyme and substrate can be held in one gel layer, to enhance the separation of the enzyme and the substrate, the coating layer may comprise two layers, one containing the substrate and the second containing the enzyme-labelled reagent. In this case, it may be preferable to incorporate into the second coating layer an agent to facilitate disruption of the liposome and hence release of the substrate. Such an agent may be a further enzyme, eg a phospholipase, or a detergent. In the latter case, non-ionic detergents are preferred since they are less likely to denature protein constituents of the system. The detergent may advantageously be one which has an insoluble structure at temperatures at which the device is stored (typically around 4°C) but which is soluble at the temperature of use (typically 37°C). Whichever lytic agent is employed, the lipid (or lipid mixture) used for the liposomes should be one that has a gel-lamellar phase transition above the device storage temperature and below the temperature of the sensor when in use. Phospholipids and detergents are generally more effective at lysing fluid lipid bilayers than their solid counterparts.

The first and second coating layers may be layers of gel, which may be of similar or different materials and may have similar or differing porosities.

Certain enzyme-substrate systems may involve two or more substrates. These different substrates may be incorporated into separate liposomes.

Examples of enzyme-substrate systems which may be employed are:

| Enzyme | Substrate 1 | Substrate 2 |
|---|---|---|
| Horse Radish Peroxidase | (1) Hydrogen Peroxide | Phenolic compounds |
| | (2) Hydrogen Peroxide | Diaminobenzidine |
| Alkaline Phosphatase | (1) 4-nitrophenylphosphate | |
| | (2) Bromochloroindoylphosphate | Nitro-blue tetrazolium |

The biosensor device according to the invention may be incorporated into various classes of biosensor. Examples are opto-electronic sensors, eg waveguide or resonant cavity devices in which the evanescent wave component is used to probe the sample. Such devices will be familiar to those skilled in the art. They can respond to either changes in refractive index or colour intensity near their active surface.

A preferred form of biosensor is one based on the principle of frustrated total reflection (FTR). The principles of FTR are well-known; the technique is described, for example, by Bosacchi and Oehrle [Applied Optics (1982), 21, 2167-2173]. An FTR device for use in immunoassay is disclosed in US Patent 4,857,273 and comprises a cavity layer bounded on one side by the sample under investigation and on the other side by a spacer layer which in turn is mounted on a substrate. The substrate-spacer layer interface is irradiated with monochromatic radiation such that total reflection occurs, the associated evanescent field penetrating through the spacer layer. If the thickness of the spacer layer is correct and the incident parallel wave vector matches one of the resonant mode propagation constants, the total reflection is frustrated and radiation is coupled into the cavity layer. The cavity layer must be composed of material which has a higher refractive index than the spacer layer and which is transparent at the wavelength of the incident radiation.

More recently, FTR biosensors have been described [see, for example, PCT Patent Applications WO 90/06503 and WO 91/06862] in which the cavity layer is a thin film of relatively high refractive index material, typically an inorganic oxide.

In practice, it may be necessary to distinguish between reaction product formed by bound (complexed) enzyme-labelled reagent and product formed by interaction with free enzyme-labelled reagent. This may be done by sensing only the region of the surface in the immediate vicinity of the device surface. For example, in a d -ice employing an evanescent field, the parameters may be chosen such that the depth of penetration of the evanescent field is relatively small. Alternatively, one may be able to rely on diffusion of the free reagents away from the surface. In a further alternative, it may be possible to provide some means of drawing a pulse of enzyme-labelled reagent over the immobilised reagent. This may be achieved, for example, by the wicking effect of, for example, a sponge gel at the sides of the active device surface.

The biosensor may be made by the steps of:
1) immobilising the first specific binding partner on the surface of the device (the methods by which this may be achieved will be known to those skilled in the art), and
2) applying the coating layer (containing the substrate, encapsulated in liposomes, and the enzyme-labelled reagent) to the s rface.

Specific embodiments of the invention will now be described, by way of illustration only, with reference to the accompanying drawings in which
Figure 1 is a schematic view of an opto-electronic biosensor,
Figure 2 is a schematic illustration of an assay of the 'sandwich' type employing the sensor of Figure 1, and
Figure 3 is a schematic illustration of a competitive assay using the sensor of Figure 1.

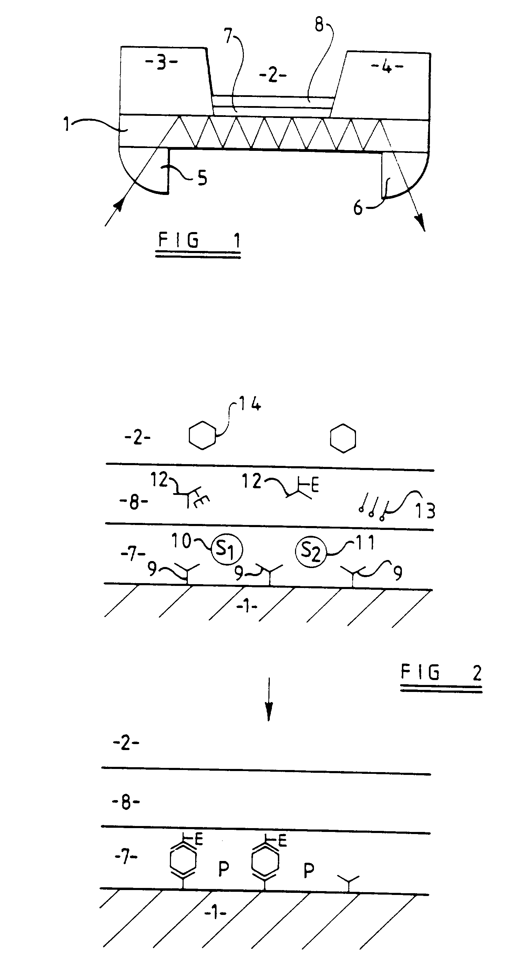

Referring first to Figure 1, an opto-electronic biosensor device comprises a quartz waveguide 1 having formed on its upper surface a sample well 2 defined by side members 3,4. Monochromatic light is coupled into the waveguide 1 via a prism 5 and out of the waveguide 1 by a second prism 6. Multiple internal reflection takes place within the waveguide 1, the evanescent wave penetrating into the sample well 2.

The surface of the waveguide 1 forming the base of the sample well 2 is coated with first and second gel layers 7,8. The first gel layer 7 includes a layer of antibody 9 immobilised on the surface of the waveguide 1 and liposomes 10,11 encapsulating two substrates S₁,S₂ of an enzyme-substrate system.

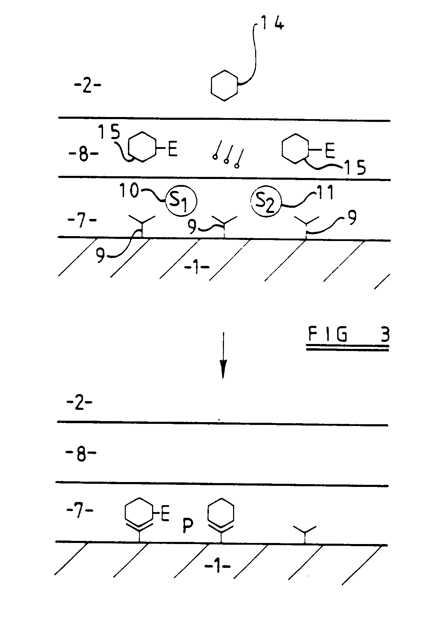

For use in a 'sandwich' type assay, as shown in Figure 2, the second gel layer 8 contains enzyme-labelled antibody 12 and a detergent 13 to disrupt the liposomes 10,11. When sample containing the antigen under test 14 is absorbed by the gel layers 7,8 a complex is formed between the enzyme-labelled antibody 12, the antigen 14 and the immobilised antibody 9. This complex formation brings the enzyme into the vicinity of the substrates S₁,S₂ and a product P is formed. Product P is strongly coloured and absorbs at the wavelength of the monochromatic light, reducing the intensity of the light coupled out of the waveguide 1 via the second prism 6. The reduction in light intensity is proportional to the analyte 14 concentration.

In the competitive assay depicted in Figure 3, the second gel layer comprises enzyme-labelled analyte 15 which competes with the antigen in the sample 14 for binding with the immobilised antibody 9. In this case, the higher the concentration of analyte 14 in the sample, the lower the concentration of enzyme reaching the vicinity of the substrates S₁, S₂ and hence the lower the concentration of product P produced. The reduction in light intensity is therefore inversely related to the analyte 14 concentration.

In an alternative format, similar to that shown in Figure 3, the second gel layer 8 comprises enzyme-labelled analyte 15 which is reversibly bound to the gel via antibodies covalently linked to the gel molecules. In this case the analyte 14 in the sample displaces the enzyme-labelled analyte 15 from the antibody in the second gel layer 8. The enzyme-labelled analyte 15 then binds to the antibody 9 immobilised in the first gel layer 7. In this case the higher the concentration of analyte 14 in the sample the higher the concentration of enzyme reaching the vicinity of the substrates S₁,S₂ and hence the higher the concentration of product P produced. The reduction in light intensity is therefore proportional to the concentration of analyte 14.

## Claims

1. A biosensor for the detection of an analyte in solution using enzyme-substrate interaction for response amplification, the biosensor having a coating layer 7,8 comprising an immobilised first specific binding partner 9 for the analyte, an enzyme-labelled reagent 12 being either a second specific binding partner for the analyte or a specific binding partner for the first specific binding partner 9, and a substrate for the enzyme, the substrate being encapsulated in a liposome 10,11.

2. A biosensor as claimed in Claim 1, wherein the liposome 10,11 is a Reversed Phase Evaporated Vesicle.

3. A biosensor as claimed in Claim 1 or Claim 2, wherein the coating layer 7,8 is a layer of gel.

4. A biosensor as claimed in Claim 3, wherein the gel is a polyacrylamide or polysaccharide gel.

5. A biosensor as claimed in any one of the preceding claims, wherein the coating layer comprises two layers 7,8, one containing the substrate and the second containing the enzyme-labelled reagent 12.

6. A biosensor as claimed in Claim 5, wherein the second coating layer 8 contains an agent 13 to facilitate disruption of the liposome 10,11 and hence release of the substrate.

7. A biosensor as claimed in any one of the preceding claims, containing two or more substrates incorporated into separate liposomes 10,11.

8. A biosensor as claimed in any one of the preceding claims, which is based on the principle of frustrated total reflection (FTR).

9. A biosensor as claimed in Claim 8, comprising a cavity layer in the form of a thin film of relatively high refractive index inorganic oxide.

10. A biosensor as claimed in any one of the preceding claims, further comprising means for drawing a pulse of enzyme-labelled reagent over the immobilised reagent.

11. A method of producing a biosensor as claimed in any one of the preceding claims, comprising the steps of:
1) immobilising the first specific binding partner 9 on the surface of the device, and
2) applying the coating layer 7,8 (containing the substrate, encapsulated in liposomes, and the enzyme-labelled reagent) to the surface.

## Patentansprüche

1. Biosensor zur Auffindung eines Analyten in Lösung, der eine Enzym-Substrat-Wechselwirkung für die Reaktionsverstärkung benutzt, wobei der Biosensor eine Beschichtung 7,8 hat, die einen immobilisierten ersten spezifischen Bindepartner 9 für den Analyten hat, ein Enzym-markiertes Reagens 12, das entweder ein zweiter spezifischer Bindepartner für den Analyten oder ein spezifischer Bindepartner für den ersten spezifischen Bindepartner 9 ist, und ein Substrat für das Enzym, wobei das Substrat in einem Liposom 10,11 eingekapselt ist.

2. Biosensor nach Anspruch 1, in dem das Liposom ein verdampftes Bläschen mit Umkehrphasen ist.

3. Biosensor nach Anspruch 1 oder Anspruch 2, in dem die Beschichtung 7,8 eine Gelschicht ist.

4. Biosensor nach Anspruch 3, in dem das Gel ein Polyacrylamid- oder ein Polysaccharidgel ist.

5. Biosensor nach einem der vorhergehenden Ansprüche, in dem die Beschichtung zwei Schichten 7,8 umfasst, von denen eine das Substrat und die andere das Enzym-markierte Reagens enthält.

6. Biosensor nach Anspruch 5, in dem die zweite Beschichtung 8 ein Mittel enthält, um Unterbrechung des Liposoms 10,11 zu ermöglichen, und daher das Substrat freizugeben.

7. Biosensor nach einem der vorhergehenden Ansprüche, der zwei oder mehr Substrate enthält, die in getrennte Liposome 10,11 eingebaut sind.

8. Biosensor nach einem der vorhergehenden Ansprüche, der auf dem Prinzip der gestörten Totalreflexion (FTR) beruht.

9. Biosensor nach Anspruch 8, der eine Hohlraumschicht in Gestalt eines dünnen Films aus anorganischem Oxid mit einem relativ hohen Brechungsindex umfasst.

10. Biosensor nach einem der vorhergehenden Ansprüche, der weiter ein Mittel umfasst, einen Puls von Enzym-markiertem Reagens über dem immoblisierten Reagens zu ziehen.

11. Verfahren zur Herstellung eines Biosensors nach einem der vorhergehenden Ansprüche, das die folgenden Schritte umfasst:
1) der erste spezifische Bindepartner 9 wird auf der Oberfläche der Einrichtung immobilisiert, und
2) die Beschichtung 7,8 (die das in Liposomen eingekapselte Substrat und das Enzym-markierte Reagens enthält) wird auf die Oberfläche aufgetragen.

## Revendications

1. Biocapteur pour la détection d'un analyte en solution utilisant l'interaction de substrat d'enzyme pour l'amplification de la réponse, le biocapteur ayant une couche de revêtement 7,8 comportant pour l'analyte un premier élément agglomérant spécifique immobilisé 9, un réactif à marquage enzyme 12 sous forme soit d'un deuxième élément agglomérant spécifique pour l'analyte ou d'un élément agglomérant spécifique pour le premier élément agglomérant spécifique 9, et un substrat pour l'enzyme, le substrat étant capsulé en liposome 10,11.

2. Biocapteur tel que revendiqué à la revendication 1, dont le liposome 10, 11 est sous forme de vésicule évaporée en phase inverse.

3. Biocapteur tel que revendiqué à la revendication 1 ou à la revendication 2, dont la couche de revêtement 7,8 est une couche de gel.

4. Biocapteur tel que revendiqué à la revendication 3, dont le gel est un gel polyacrylamide ou polysaccharide.

5. Biocapteur tel que revendiqué à l'une quelconque des revendications précédentes, dont la couche de revêtement comporte deux couches 7,8, dont l'une contenant le substrat et la deuxième contenant le réactif à marquage enzyme 12.

6. Biocapteur tel que revendiqué à la revendication 5, dont la deuxième couche de revêtement 8 contient un réactif pour faciliter la rupture du liposome 10,11, et par conséquent la libération du substrat.

7. Biocapteur tel que revendiqué à l'une quelconque des revendications précédentes, contenant deux ou plusieurs substrats incorporés à des liposomes séparés 10,11.

8. Biocapteur tel que revendiqué à l'une quelconque des revendications précédentes, basé sur le principe de la réflection totale frustrée (RTF).

9. Biocapteur tel que revendiqué à la revendication 8, comportant une couche creuse sous forme de fine pellicule d'oxyde inorganique d'indice réfractif relativement élevé.

10. Biocapteur tel que revendiqué à l'une quelconque des revendications précédentes, comportant en outre des moyens pour tirer une impulsion de réactif à marquage enzyme sur le réactif immobilisé.

11. Méthode de production de biocapteur tel que revendiqué à l'une quelconque des revendications précédentes, comportant les phases suivantes:
1) immobilisation du premier élément agglomérant spécifique 9 en surface du dispositif, et
2) application en surface de la couche de revêtement 7,8 (contenant le substrat capsulé sous liposomes, et le réactif à marquage enzyme).
